(19) Europäisches Patentamt
European Patent Office
Office européen des brevets

(11) Veröffentlichungsnummer: **0 153 642**
**A2**

(12) # EUROPÄISCHE PATENTANMELDUNG

(21) Anmeldenummer: 85101413.4

(22) Anmeldetag: 11.02.85

(51) Int. Cl.⁴: **C 07 C 125/065**

(30) Priorität: 21.02.84 DE 3406230

(43) Veröffentlichungstag der Anmeldung:
04.09.85 Patentblatt 85/36

(84) Benannte Vertragsstaaten:
AT BE CH DE FR GB IT LI NL

(71) Anmelder: BAYER AG
Konzernverwaltung RP Patentabteilung
D-5090 Leverkusen 1 Bayerwerk(DE)

(72) Erfinder: Stammann, Günter, Dr.
Silesiusstrasse 78
D-5000 Koeln(DE)

(72) Erfinder: Grolig, Johann, Dr.
Heinrich-Lübke-Strasse 22
D-5090 Leverkusen 1(DE)

(72) Erfinder: Waldmann, Helmut, Dr.
Henry T. v. Böttinger Strasse 15
D-5090 Leverkusen 1(DE)

(54) **Verfahren zur Herstellung monomerer aromatischer Hydroxyurethane.**

(57) Monomere aromatische Hydroxyurethane werden hergestellt, indem man eine aromatische Verbindung, die pro Molekül mindestens eine direkt an einen aromatischen Kern gebundene Hydroxylgruppe und mindestens eine Nitro-, Nitroso-, Azo- oder Azoxygruppe enthält mit einer organischen Hydroxyverbindung und Kohlenmonoxid in Gegenwart spezieller Katalysatoren umsetzt.

EP 0 153 642 A2

0153642

BAYER AKTIENGESELLSCHAFT 5090 Leverkusen, Bayerwerk
Konzernverwaltung RP Gai/by-c
Patentabteilung

Verfahren zur Herstellung monomerer aromatischer Hydroxyurethane

Die vorliegende Erfindung betrifft ein neues Verfahren zur Herstellung von monomeren aromatischen Hydroxyurethanen aus aromatischen Verbindungen, die mit Hydroxylgruppen und Stickstoff enthaltenden Gruppen substituiert sind.

Zur Herstellung von monomeren aromatischen Hydroxyurethanen, die auch als Hydroxycarbamate bezeichnet werden, sind mehrstufige Verfahren bekannt, bei denen zunächst Hydroxynitroverbindungen zu Aminohydroxyverbindungen reduziert werden, beispielsweise werden durch Hydrierung von Nitrophenolen Aminophenole erhalten (siehe z.B. Kirk-Othmer, Encyclopedia of Chemical Technology, Vol. II, New York 1978, S. 422 ff). Die Aminophenole werden dann entweder durch Phosgenierung in die Hydroxyisocyanate überführt, aus denen sich durch Umsetzung mit Alkoholen aromatische Hydroxyurethane bilden, oder sie werden mit den aus Phosgen und Alkoholen zugänglichen Chlorameisensäurealkylestern zur Reaktion gebracht und liefern so neben

0153642

anderen Produkten aromatische Hydroxyurethane (siehe z.B. US-PS 3 852 332, US-PS 3 933 470 und P. Chabrier, H. Najer und R. Gindicelli, Bull, Soc. Chim. France (1955), S. 1353 ff.).

Neben der Mehrstufigkeit der bisher bekannten Verfahren zur Herstellung monomerer aromatischer Hydroxyurethane und der Nebenproduktbildung sind die geringe Lagerbeständigkeit und die schwierige Handhabung der Zwischenprodukte, die insbesondere die Herstellung im technischen Maßstab sehr erschwert, nachteilig.

Weiterhin sind Verfahren zur Herstellung von monomeren Urethanen bekannt, bei denen man Nitroverbindungen, Alkohole und Kohlenmonoxid in Gegenwart von Katalysatoren miteinander umsetzt. Die einzusetzenden Nitroverbindungen können auf vielfältige Weise substituiert sein, jedoch nicht mit Hydroxygruppen. Somit können auf diese Weise auch substituierte Urethane hergestellt werden (siehe z.B. US-PS 3 454 620, DE-OS 2 903 950, DE-OS 2 838 754, DE-OS 2 808 980, EP-OS 0 086 281, EP-OS 0 083 096 und EP-OS 0 029 460).

In der EP-OS 0 000 563 wird die Herstellung monomerer aromatischer Urethane aus aromatischen Nitroverbindungen, Alkoholen und Kohlenmonoxid in Gegenwart von Katalysatoren und bestimmten Stickstoffverbindungen beschrieben. Als einsetzbare Alkohole werden u.a. aromatische Hydroxynitroverbindungen genannt (siehe Seite 5, Zeilen 11 bis 23). Somit lehrt diese Offenlegungsschrift,

daß aromatische Hydroxynitroverbindungen bei der Urethanbildung mit ihrer Hydroxygruppe reagieren und so den O-Substituenten eines N-O-disubstituierten Urethans ergeben.

Gemäß US-PS 3 956 360 und US-PS 3 993 685 können Hydroxynitroverbindungen mit Kohlenmonoxid in Gegenwart von Katalysatoren zu Urethanen umgesetzt werden. In diesen Fällen reagieren die Nitrogruppen und die Hydroxylgruppen der Hydroxynitroverbindungen und es werden lineare Polyurethane erhalten (siehe insbesondere US-PS 3 956 360, Spalte 9, Zeilen 18 bis 22).

Die Herstellung von Hydroxyurethanen aus Nitrophenolen oder sonstigen aromatischen Verbindungen, die pro Molekül mindestens eine direkt an einen aromatischen Kern gebundene Hydroxylgruppe und mindestens eine Nitro-, Nitroso-, Azo- oder Azoxygruppe enthalten und organischen Hydroxyverbindungen durch eine Carbonylierungsreaktion ist aus diesen Literaturstellen also nicht bekannt. Gemäß dem vorstehend beschriebenen Stand der Technik ist zu erwarten, daß bei einer solchen Reaktion entweder polymere Urethane entstehen oder die Hydroxygruppe am Nitroaromaten wie eine Alkoholkomponente reagiert.

In der EP-OS 94 861 wird ein Verfahren zur Herstellung von Polyurethanen beschrieben, bei dem in flüssiger Phase im allgemeinen in Gegenwart eines Lösungsmittels, para- oder meta-Nitro-Hydroxyaromaten mit Kohlenmonoxid umgesetzt werden in Gegenwart von Katalysatoren auf der Basis von Edelmetallen der Gruppe VIII, die gegebenenfalls ein zweites Übergangsmetall aus den Gruppen Vb, VIb oder VIII enthalten können. Heteroaromatische Basen können auch zugegen sein.

Bei dem zweiten Übergangsmetall kann es sich um Molybdän, Vanadin oder Eisen in metallischer oder oxidischer Form handeln. Wenn man dieses Verfahren in Gegenwart eines monofunktionellen Alkohols durchführt, bricht, in Abhängigkeit von der Alkoholmenge, die Polyurethankette auf und es bilden sich Carbamatgruppen. Bei einem Überschuß des Alkohols kann man keine Polyurethane mehr isolieren, sondern monomere Carbamate. Aus diesen kann man durch Erhitzen, vorzugsweise in Gegenwart eines Katalysators wie Zink- oder Eisenchlorid, das polymere Polyurethan wiedergewinnen. Nachteilig bei diesem Verfahren ist, daß nur geringe Raum-Zeit-Ausbeuten an Carbamaten (= monomeren Hydroxyurethanen) erzielt werden können, relativ große Mengen an Edelmetall-Katalysatoren einzusetzen sind und größere Edelmetallverluste auftreten.

Es wurde nun ein Verfahren zur Herstellung monomerer aromatischer Hydroxyurethane gefunden, das dadurch gekennzeichnet ist, daß man eine aromatische Verbindung, die pro Molekül mindestens eine direkt an einen aromatischen Kern gebundene Hydroxylgruppe und mindestens eine Nitro-, Nitroso-, Azo- oder Azoxygruppe enthält mit einer organischen Hydroxyverbindung und Kohlenmonoxid in Gegenwart eines Katalysators umsetzt, der a) Schwefel und/oder Selen oder b) Edelmetalle der 8. Nebengruppe des Periodensystems der Elemente in elementarer und/oder in Form von Verbindungen, eine oder mehrere als Komplexligand geeignete Stickstoff- und/oder Phosphorverbindungen und eines oder mehrere aktivierende Halogenide enthält.

In das erfindungsgemäße Verfahren zur Herstellung von monomeren aromatischen Hydroxyurethanen (= Hydroxycarbamaten) werden demnach mindestens vier verschiedene Arten von Stoffen eingesetzt, nämlich, eine aromatische

Verbindung, die pro Molekül mindestens eine direkt an einen aromatischen Ring gebundene Hydroxylgruppe und mindestens eine Nitro-, Nitroso-, Azo- oder Azoxygruppe enthält (im folgenden als Verbindung A bezeichnet), eine organische Hydroxyverbindung, Kohlenmonoxid und ein Katalysator.

Bei der Verbindung A kann es sich beispielsweise um eine Nitro- oder Nitrosoverbindung der Formel (I) oder um eine Azo- oder Azoxyverbindung der Formel (II)

$$(HO)_a Z (NO_x)_b \qquad (I)$$

$$(HO)_a \underset{(NO_x)_c}{Z} - [\bar{N} = \overset{(O)_d}{N} - R_1 - ]_b \qquad (II)$$

handeln, in denen

Z für einen insgesamt 4 bis 30 C-Atome enthaltenden Rest steht, der mindestens einen aromatischen Ring enthält und der gegebenenfalls weitere als die in den Formeln (I) und (II) angegebenen Substituenten enthält,

x für 1 oder 2 steht,

a und b unabhängig voneinander für 1, 2, 3 oder 4 stehen,

c für Null, 1, 2 oder 3 steht,

die Summe a + b + c jedoch nicht größer ist, als die um 2 verminderte Anzahl von substituierbaren Stellen an Z

d für Null oder 1 steht,

$R_1$ für einen aliphatischen, cycloaliphatischen oder aromatischen Rest mit insgesamt 1 bis 30 C-Atomen steht, der gegebenenfalls substituiert sein kann,

und wobei mindestens eine HO-Gruppe direkt an einen aromatischen Ring von Z gebunden ist.

In den Formeln (I) und (II) kann Z beispielsweise einen oder mehrere, beispielsweise 1 bis 3, carbocyclische und/oder heterocyclische aromatische Ringe enthalten.

Wenn mehrere aromatische Ringe vorliegen können diese anelliert und/oder über C- und/oder über Heteroatome miteinander verknüpft sein, beispielsweise über Kohlenwasserstoff-, Keto-, Ether-, Ester-, Amino-, Alkylamino-, Harnstoff-, Urethan-, Phosphorester-, Thio-, Thioester-, Thioharnstoff-, Thioether- und/oder Thiophosphorestergruppen. Aromatische Ringsysteme, die in Z enthalten sein können, können unabhängig voneinander beispielsweise das Grundgerüst von Benzol, Indan, Inden, Naphthalin, Anthracen, Phenanthren, Acenaphten, Furan, Thiophen, Pyrrol, Pyrazol, Oxazol, Imidazol, Pyrimidin, Chinolin, Benzimidazol, Indol, Carbazol und Phenazin enthalten.

Die in Z enthaltenen aromatischen Ringe können zusätzlich zu den in den Formeln (I) und (II) angegebenen Substituenten weitere Substituenten enthalten.

Beispiele für solche Substituenten sind Alkylgruppen mit beispielsweise 1 bis 10 C-Atomen, wie Methyl, Ethyl, Propyl, Butyl, Pentyl, Hexyl, Octyl und Decyl; Cycloalkylgruppen mit beispielsweise 5 bis 10 C-Atomen, wie Cyclopentyl, Cyclohexyl und Cycloheptyl; Halogenatome, wie Fluor, Chlor, Brom und Iod; Pseudohalogengruppen, wie Cyano; Aldehydgruppen; Sulfogruppen; Carboxyalkylgruppen mit beispielsweise 1 bis 10 C-Atomen, wie Carboxymethyl, Carboxyethyl, Carboxypropyl und Carboxybutyl; Acylgruppen mit beispielsweise 1 bis 10 C-Atomen, wie Acetyl, Propionyl, Butyryl, Valeroyl und Caproyl; Aminogruppen; N-Alkylaminogruppen mit beispielsweise 1 bis 10 C-Atomen wie N-Methyl-, N-Ethyl-, N-Propyl-, N-Butyl-, N-Pentyl- und N-Hexylamino; N,N-Dialkylaminogruppen mit beispielsweise 1 bis 10 C-Atomen, wie N,N-Dimethyl-, N,N-Methylethyl-, N,N-Diethyl-, N,N-Methylpropyl-, N,N-Ethylpropyl-, N,N-Dipropyl- und N,N-Dibutylamino; N-Acylaminogruppen mit beispielsweise 1 bis 10 C-Atomen, wie N-Acetylamino, N-(Phenylacetyl)-amino, N-Benzoylamino, N-Methyl-N-acetylamino und N-4-Chlorbenzoylamino; Harnstoffgruppen; Phosphorestergruppen; Phosphorthioestergruppen; Ethergruppen mit beispielsweise 1 bis 10 C-Atomen, wie Methoxy, Ethoxy, Propoxy, Isopropoxy, Butoxy und Phenoxy; entsprechende Thioethergruppen und nichtaromatische heterocyclische Reste mit beispielsweise 4 bis 10 C-Atomen, wie Pyrrolidinyl, Piperidinyl, Morpholinyl und Chinuclidinyl.

Wenn als Substituenten Amino- und/oder Carboxygruppen vorhanden sind können diese unter den Reaktionsbedingungen alkyliert werden. Man erhält dann die entsprechenden N-Alkylamine bzw. Carbonsäurealkylester. Wenn als Substituenten Harnstoffgruppen vorhanden sind, können diese sich während des erfindungsgemäßen Verfahrens verändern.

Bevorzugte Substituenten sind Chlor, Fluor, Methyl, Methoxy, Ethoxy, Isopropoxy, N,N-Dimethylamino, N-Acetylamino und Carboxy-$C_1$-$C_4$-alkyl.

Die oben beschriebenen Substituenten können nicht nur direkt an in Z enthaltenen aromatischen Ringen, sondern auch an Brücken zwischen solchen aromatischen Ringen und/oder an Seitenketten solcher aromatischer Ringe gebunden sein.

Vorzugsweise steht Z für einen Rest, der das Grundgerüst von Benzol, Naphthalin oder Pyridin als Grundstruktur enthält. Diese Reste können gegebenenfalls unabhängig voneinander mit bis zu 4 gleichen oder verschiedenen Substituenten der oben beschriebenen Art substituiert sein, insbesondere mit den als bevorzugt beschriebenen Substituenten.

Besonders bevorzugt steht Z für einen Rest, der das Grundgerüst des Benzols enthält und der gegebenenfalls mit bis zu 2 Substituenten substituiert sein kann, insbesondere mit den als bevorzugt bezeichneten Substituenten.

In den Formeln (I) und (II) steht x vorzugsweise für 2, d.h. die Nitroverbindungen der Formeln (I) und (II) sind gegenüber den entsprechenden Nitrosoverbindungen bevorzugt.

In den Formeln (I) und (II) steht a vorzugsweise für 1, 2 oder 3. Mindestens eine Hydroxygruppe ist dabei direkt an einen aromatischen Ring in Z gebunden. Weitere Hydroxygruppen können auch an Seitenketten und/oder an Brücken zwischen aromatischen Ringen gebunden sein. Vorzugsweise sind alle vorhandenen Hydroxygruppen direkt an aromatische Ringe in Z gebunden.

Besonders bevorzugt steht a in den Formeln (I) und (II) für 1 oder 2, ganz besonders bevorzugt für 1.

Wenn in den Formeln (I) und (II) b für 3 oder 4 steht sind vorzugsweise an einen aromatischen Ring in Z höchstens 2 Nitro-, Nitroso-, Azo- oder Azoxygruppen gebunden. Besonders bevorzugt steht b für 1. Die Nitro-, Nitroso-, Azo- und Azoxygruppen können direkt an aromatische Ringe in Z, aber auch an Seitenketten und/oder an Brücken zwischen aromatischen Ringen gebunden sein. Vorzugsweise sind die Nitro-, Nitroso-, Azo- und Azoxygruppen direkt an aromatische Ringe in Z gebunden.

Die von den Formeln (I) und (II) umfaßten Nitro- und Nitrosoverbindungen enthalten, wenn die Nitro- oder Nitrosogruppen an aromatische Ringe gebunden sind, in ortho-Stellung dazu vorzugsweise keine weiteren Nitro- oder Nitrosogruppen und keine Hydroxy- oder Aminosubstituenten.

In der Formel (II) steht c vorzugsweise für Null oder 1, besonders bevorzugt für Null, d.h. bevorzugte Azo- und Azoxyverbindungen der Formel (II) enthalten höchstens eine, besonders bevorzugt keine Nitro- oder Nitrosogruppen.

In der Formel (II) steht d vorzugsweise für Null, d.h. die Azoverbindungen der Formel (II) sind gegenüber den entsprechenden Azoxyverbindungen bevorzugt.

Sofern in Formel (II) $R_1$ für einen substituierten aliphatischen, cycloaliphatischen oder aromatischen Rest steht, können daran als Substituenten beispielsweise OH-, $NO_2$- und/oder NO-Gruppen vorhanden sein, sowie die

Substituenten, die weiter oben als weitere Substituenten für die in Z enthaltenen aromatischen Ringe beschrieben sind. Vorzugsweise ist $R_1$ ein 5 bis 30 C-Atome enthaltender Rest, der mindestens einen aromatischen Ring enthält. Besonders bevorzugt entspricht in Formel (II) der Rest $R_1$ dem jeweils vorhandenen Molekülteil $(HO)_a-\underset{(NO_x)_c}{Z}-$, d.h. besonders bevorzugt sind solche Azo- und Azoxyverbindungen, die an den beiden Stickstoffatomen die gleichen Substituenten aufweisen.

Die Verbindungen der Formel (I) sind gegenüber den Verbindungen der Formel (II) bevorzugt.

Geeignete Verbindungen A sind beispielsweise 4,4'-Dihydroxyazobenzol, p,p'-Azoxyphenol, 4-[2-Methyl-2-(4-nitrophenyl)-ethyl]-phenol, 1-Nitro-2-(4-hydroxyphenyl)-ethan, 2-Nitrophenol, 3-Nitrophenol, 4-Nitrophenol, 4-Nitrosophenol, 3-Nitrosophenol, 2-Nitro-1-naphthol, 3-Nitro-1-naphthol, 4-Nitro-1-naphthol, 5-Nitro-1-naphthol, 1-Nitro-2-naphthol, 5-Nitro-2-naphthol, 6-Nitro-2-naphthol, 8-Nitro-2-naphthol, 4-Nitroso-naphthol, 2-Nitro-2'-hydroxy-diphenyl, 2-Nitro-4'-hydroxy-diphenyl, 4-Nitro-4'-hydroxydiphenyl, 2-Nitro-4'-hydroxybenzophenon, 2-Nitroanthranol-9, 4-Nitroanthranol-1, 2-Hydroxy-8-nitrochinolin, 1-Nitro-4-hydroxy-phenthiazin, 1-Nitro-6-hydroxy-phenazin, 3-Nitro-5-(2-pyridyl)-phenol, 4-Nitro-2-(2-furyl)-phenol, 2-Chlor-4-nitrophenol, 3-Chlor-4-nitrophenol, 4-Chlor-2-nitrophenol, 2-Chlor-3-nitrophenol, 4-Chlor-3-nitrophenol, 5-Chlor-3-nitrophenol, 2-Chlor-5-nitrophenol, 2-Brom-4-nitrophenol, 3-Fluor-4-nitrophenol, 4-Iod-3-nitrophenol, die isomeren Dichlor- und Trichlornitrophenole, gemischt halogenierte Nitrophenole, halogenierte Nitronaphthole, arylsubstituierte Nitrophenole, beispielsweise 2-Phenyl-4-nitrophenol, Alkyl- oder Cycloalkyl-substituierte Nitrophenole, beispielsweise 4-Methyl-3-nitrophenol, 2-tert.-Butyl-4-nitrophenol, 3-Methyl-4-nitrophenol oder 3-Cyclohexyl-5-nitrophenol, Alkoxy- oder Phenoxy-substituierte Nitrophenole, beispielsweise 2-Methoxy-3-nitrophenol, 5-Ethoxy-3-nitrophenol, 3-Isopropoxy-4-nitrophenol, 5-Nitro-5-phenoxyphenol, 5-Nitrosalicylsäureethylester und 5-Nitrosalicylaldehyd, ferner 2-Chlor-6-methyl-4-nitrophenol, 2-Isopropoxy-4-chlor-5-nitrophenol, 5-tert.-Butyl-3-nitro-1-naphthol, 1-Methoxy-4-nitro-2-naphthol, 3-(4-Fluorphenyl)-5-nitrophenol, N-(2-Chlor-4-nitrophenyl)-5-chlor-2-hydroxy-benzoesäureamid, 3-Hydroxymethyl-5-nitrophenol, 4-Nitrobenzcatechin, 5-Nitroresorcin, sym. Nitropyrogallol, 1,5-Dihydroxy-3-nitronaphthalin, 4,6-Dinitroo-kresol, 2,4-Dinitronaphthol-1, 3,4-Dinitrophenol, 2,4-Dinitrophenol, 3,5-Dinitrophenol und 2,2'-Dihydroxy-4,4'-dinitrobiphenyl.

Bevorzugte Verbindungen A sind 3-Nitrophenol, 4-Nitrophenol, 4-Nitrosophenol, 4-Nitrosonaphthol-1, 4-Nitronaphthol-1, 5-Nitronaphthol-1, 5-Nitronaphthol-2, 6-Nitronaphthol-2, 8-Nitronaphthol-2 und Derivate dieser Verbindungen mit Substituenten aus der Gruppe Fluor,

Chlor, Brom, $C_1$-$C_4$-Alkyl, $C_5$-$C_7$-Cycloalkyl, $C_7$-$C_{12}$-Aralkyl, Phenyl, $C_1$-$C_4$-Alkoxyl, Phenoxyl sowie 4-Nitrobrenzcatechin, 5-Nitroresorcin und sym. Nitropyrogallol.

Besonders bevorzugte Verbindungen A sind 3-Nitrophenol und 4-Nitrophenol sowie deren mit Chlor, Fluor, Methyl, Methoxy, Ethoxy, Isopropoxy, N,N-Dimethylamino, N-Acetylamino oder Carboxy-$C_1$ bis $C_4$-alkyl substituierten Derivate.

In das erfindungsgemäße Verfahren können als organische Hydroxyverbindungen die verschiedensten Alkohole und Phenole eingesetzt werden, beispielsweise solche der Formel (III)

$$R_2-(OH)_n \qquad (III)$$

in der

$R_2$ für einen gegebenenfalls substituierten organischen Rest mit insgesamt 1 bis 30 C-Atomen und

n für 1, 2, 3 oder 4 steht.

$R_2$ kann beispielsweise ein Alkyl-, Cycloalkyl-, Aryl- oder Aralkylrest sein, der gegebenenfalls substituiert sein kann, beispielsweise durch solche Substituenten, wie sie weiter oben als weitere Substituenten für Z beschrieben sind. Sofern $R_2$ carbocyclische und/oder heterocyclische Strukturelemente enthält können diese isoliert, anelliert und/oder über C- und/oder Heteroatome miteinander verknüpft sein.

In Formel (III) steht $R_2$ vorzugsweise für einen gegebenenfalls substituierten organischen Rest mit insgesamt 1 bis 12 C-Atomen und n vorzugsweise für 1 oder 2.

Besonders bevorzugt steht in Formel (III) $R_2$ für einen gegebenenfalls substituierten aliphatischen oder cycloaliphatischen Rest mit insgesamt 1 bis 6 C-Atomen und n für 1.

Ganz besonders bevorzugt steht in Formel (III) $R_2$ für einen unsubstituierten primären oder sekundären Rest mit 1 bis 6 C-Atomen und n für 1.

Beispiele für in das erfindungsgemäße Verfahren einzusetzende organische Hydroxyverbindungen sind Methanol, Ethanol, 1-Propanol, 2-Propanol, 1-Butanol, 2-Butanol, tert.-Butanol, die isomeren Pentanole, insbesondere 2,2-Dimethyl-propanol, die isomeren Hexanole, höhere Fettalkohole und Fettalkoholgemische, 2-Propen-1-ol, 3-Butin-2-ol, Glykol, Butan-1,4-diol, 2-Methyl-2,4-pentandiol, 2-Ethyl-2-hydroxymethyl-1,3-propandiol, Pentaerythrit, Benzylalkohol, p-Hydroxybenzylalkohol, Hydroxybenzylalkohol, Cyclohexanol, 2-Methylcyclohexanol, 3,3,5-Trimethylcyclohexanol, 2-Hydroxydecalin, 2-Phenylethanol, Furfurylalkohol, 1,2,3,4-Tetrahydro-1-naphthol, 1,4-Cyclohexandiol, 1,4-Bishydroxymethylcyclohexan, Bishydroxymethylhexahydro-4,7-methanoindan (TCD-diol), 2-Chlorethanol, 1,3-Dichlor-2-propanol, Methoxyethanol, 2,2'-Dihydroxydiethylether, Diethylenglykolmonomethylether u.ä., 2-Diethylaminoethanol, 4-Methoxybenzylalkohol, 4-Fluorbenzylalkohol und 3-Hydroxypropannitril, ferner Phenol, 1-Naphthol, 2-Naphthol, 4-Hydroxypyridin, 2-Hydroxyphenazin, 2-Chlorphenol, 3-Chlorphenol, 4-Chlorphenol, Di- und Trichlorphenole, 4-Fluorphenol, Resorcinmonomethylether, 4-Chlor-3-methylphenol und 4-Phenylphenol.

Bevorzugte in das erfindungsgemäße Verfahren einzusetzende organische Hydroxyverbindungen sind gesättigte aliphatische und cycloaliphatische Mono- und Dialkohole mit bis zu 12 C-Atomen, insbesondere Methanol, Ethanol, 1-Propanol, 2-Propanol, 1-Butanol, 2,2-Dimethylpropanol und 2,2,3-Trimethyl-1-butanol.

Im erfindungsgemäßen Verfahren reagieren die Hydroxygruppen der eingesetzten organischen Hydroxyverbindungen mit den Verbindungen A und Kohlenmonoxid unter Ausbildung von Hydroxyurethangruppen. Bei organischen Hydroxyverbindungen, die pro Molekül mehr als eine OH-Gruppe enthalten, können je nach der Reaktivität der OH-Gruppen und dem angewendeten Molverhältnis der Verbindung A zur organischen Hydroxyverbindung, eine oder mehrere OH-Gruppen in dieser Weise reagieren.

Im allgemeinen setzt man die organische Hydroxyverbindung, bezogen auf die Verbindung A, im großen molaren Überschuß ein, beispielsweise im Molverhältnis von 3:1 bis 100:1. Wenn es sich

bei der organischen Hydroxyverbindung um ein teures oder unter Reaktionsbedingungen zu thermischer Zersetzung neigendes Produkt handelt oder wenn organische Hydroxyverbindungen, die mehrere OH-Gruppe enthalten, an mehreren OH-Gruppen zur Hydroxyurethanbildung gebracht werden sollen, ist es zweckmäßig, die organische Hydroxyverbindung in geringerer Menge einzusetzen, beispielsweise in der 0,5- bis 1,5-fachen molaren Menge, die theoretisch notwendig ist, um die Verbindung A in der gewünschten Weise reagieren zu lassen. In diesen Fällen ist es dann vorteilhaft, zusätzlich ein inertes Lösungsmittel einzusetzen, beispielsweise einen Kohlenwasserstoff, einen chlorierten Kohlenwasserstoff, ein Keton oder einen Ether. Hierfür geeignet sind beispielsweise Toluol, Methylenchlorid, Furan, Dioxan, Aceton, Chlorbenzol und/oder ortho-Dichlorbenzol. Im allgemeinen wird jedoch ohne Zusatz von Fremdlösungsmittel gearbeitet.

Kohlenmonoxid kann in das erfindungsgemäße Verfahren in reiner Form oder in Form eines Kohlenmonoxid enthaltenden Gases eingesetzt werden. Geeignetes Kohlenmonoxid kann nach verschiedenen technischen Verfahren, z.B. durch Reformieren oder Vergasungsprozesse und nachgeschaltete Reinigungsstufen erhalten werden. Als Verunreinigungen können im Kohlenmonoxid Verbindungen enthalten sein, die sich im erfindungsgemäßen Verfahren vollständig oder weitgehend inert verhalten, beispielsweise Stickstoff, Helium, Argon, Kohlendioxid und/oder niedere Kohlenwasserstoffe, insbesondere Methan. Für die technische Durchführung des erfindungsgemäßen Verfahrens kann es vorteilhaft sein, dem Kohlenmonoxid Inertgase zuzumischen, vorzugsweise Stickstoff und/oder

Kohlendioxid, die insgesamt mit bis zu 70 Vol.-% im Kohlenmonoxid enthaltenden Gas vorliegen können.

Schwefelhaltige Verunreinigungen des Kohlenmonoxids, insbesondere Schwefelwasserstoff und COS, sollten bei Verwendung auf Edelmetallen basierender Katalysatorsysteme nach üblichen technischen Verfahren praktisch vollständig, d.h. auf maximal 1 ppm Schwefel entfernt werden, um Vergiftungen des Katalysators zu vermeiden. Sollen auf Schwefel oder Selen basierende Katalysatorsysteme angewendet werden, so brauchen evtl. im Kohlenmonoxid vorhandene schwefelhaltige Verunreinigungen nicht entfernt zu werden, da diese das Katalysatorsystem ergänzen oder als Katalysatorkomponente dienen können.

Als weitere Verunreinigung kann in Kohlenmonoxid Wasserstoff zugegen sein. Dieser kann beschleunigend oder reduzierend in die Urethanbildungsreaktion eingreifen. Um Reinigungskosten bei der Kohlenmonoxidherstellung einzusparen kann es vorteilhaft sein, bis zu 5 Vol.-% Wasserstoff enthaltendes Kohlenmonoxid einzusetzen, insbesondere wenn das Kohlenmonoxid aus einem Synthesegas nach dem Membrantrennverfahren gewonnen wurde. Höhere Wasserstoffanteile sind zwar möglich, doch ohne besondere Vorteile für das erfindungsgemäße Verfahren. Ebenso kann die Zugabe geringer Ammoniakmengen, z.B. bis zu 5 Vol.-%, zum Reaktionsgas die Reaktionsgeschwindigkeit beim erfindungsgemäßen Verfahren erhöhen.

Das Kohlenmonoxid kann im Unterschuß oder im Überschuß der Stöchiometrie, nach welcher z.B. für die Umsetzung

einer Nitrogruppe 3 Moleküle CO benötigt werden, eingesetzt werden. Bevorzugt wird es in einem Überschuß von 5 bis 1000 Mol-%, besonders bevorzugt in einem Überschuß von 5 bis 200 Mol-% verwendet.

Bei den in das erfindungsgemäße Verfahren einzusetzenden Katalysatoren kann es sich um solche handeln, die an sich für die Urethanbildung aus Nitroverbindungen, Alkoholen und Kohlenmonoxid bekannt sind. Die Katalysatoren bzw. Katalysatorsysteme enthalten entweder a) Schwefel und/oder Selen oder b) Edelmetalle der 8. Nebengruppe des Periodensystems der Elemente (nach Mendelejew) in elementarer Form und/oder in Form von Verbindungen sowie eine oder mehrere als Komplexligand geeignete Stickstoff- und/oder Phosphorverbindungen und eines oder mehrere aktivierende Halogenide.

In speziellen Ausführungsformen können die Katalysatorsysteme der Arten a) und b) weitere Komponenten enthalten, z.B. können die Katalysatorsysteme a) auch Verbindungen enthalten, die als Komplexliganden des Katalysatortyps b) angegeben sind.

Katalysatoren der Art a) enthalten beispielsweise Schwefel in beliebiger Form, Carbonylsulfid (COS), Schwefelwasserstoff, Alkalisulfide, Dimethylsulfid, Thiophen und Thioharnstoff sowie Selen in beliebiger Form, vorzugsweise metallisches Selen, Selendioxid, Carbonylselenid, Dimethylselenid und Diphenylselenid. Katalysatoren der Art b) können als Edelmetallkomponente beispielsweise Ruthenium, Rhodium, Palladium, Osmium, Iridium und/oder Platin oder Verbindungen dieser Edelmetalle enthalten. Katalysatoren der Art b) sind gegenüber Katalysatoren der Art a) bevorzugt.

Die als Komplexliganden geeigneten Stickstoff- und/oder Phosphorverbindungen sind eine wesentliche Komponente von Katalysatoren der Art b) und eine wahlweise Komponente von Katalysatoren der Art a). Es kann sich hierbei um primäre, sekundäre oder tertiäre Amine handeln, die linear oder in Form von Stickstoff-Heterocyclen vorliegen können, sowie um Harnstoffe, Biurete und substituierte Phosphine und Phosphite. Auch polymere Amine, z.B. Polyvinylpyridin oder pyrolysiertes Polyacrylnitril sowie polymer gebundene Phosphine, beispielsweise solche gemäß US 3 708 462, sind geeignet. Ebenso können Chelatbildner mit mehr als einer zur Koordination befähigten N- bzw. P-Funktion eingesetzt werden. Amine und Phosphine können auch in Form ihrer Hydrochloride und/oder in Form von Metallsalzkomplexen eingebracht werden, soweit diese ausreichend beständig sind.

Eine weitere wesentliche Komponente von Katalysatoren der Art b) sind aktivierende Halogenide. Es kann sich hierbei beispielsweise um Halogenide und/oder Oxyhalogenide von solchen Metallen handeln, die in mehreren Wertigkeitsstufen auftreten können. Bevorzugt sind die Chloride bzw. Chloridhydrate und Oxychloride solcher Metalle, beispielsweise $FeCl_2$, $FeCl_3$, $NiCl_2$, $CoCl_2$, $CuCl_2$, $VCl_5$, $FeOCl$ und $VOCl_3$. Lithiumchlorid, Ammoniumchlorid und substituierte Ammoniumchloride, die bis zu vier organische Substituenten tragen, sowie Chlorwasserstoff sind ebenfalls geeignete Halogenide. Chlorwasserstoff kann z.B. in Form der Hydrochloride der oben beschriebenen Amine eingesetzt werden.

Besonders geeignete Katalysatorsysteme enthalten Palladium oder Ruthenium in metallischer Form oder als Oxide, Chloride, Bromide, Sulfate, Nitrate, Acetate oder Acetylacetonate und ein tertiäres Amin oder ein tertiäres

Phosphin aus der Gruppe Triethylamin, Tributylamin, N,N'-Tetramethylethylendiamin-1,2, N,N'-Diethylanilin, Cyclohexyldimethylamin, Pyridin, Picoline, Lutidine, Chinolin, Imidazol, Benzimidazol, 1,4-Diazabicyclo(2,2,2)-octan, 1,8-Diazabicyclo(5,4,0)-undecen-7, Pyrazol, 2,2'-Bipyridin, Ethylen-bis-(salicylimin), 1,10-Phenanthrolin, N,N,N',N'-Tetramethyl-o-phenylendiamin, 1,4,8,11-Tetramethyl-1,4,8,11-tetraazacyclotetradecan, Triphenylphosphin, Tri-n-propylphosphin, Bis-(1,3-diphenylphosphino)-propan, Tris-(4-dimethylaminophenyl)-phosphin und Bis-(2-cyanoethyl)-phenylphosphin.

Für Katalysatoren der Art b) können beispielsweise Edelmetalle oder Edelmetallverbindungen in Mengen von 0,0001 bis 0,02 Gew.-%, bevorzugt in Mengen von 0,0001 bis 0,01 Gew.-% eingesetzt werden, jeweils berechnet als Edelmetall und hier wie im folgenden bezogen auf die Gesamtmenge der bei Normalbedingungen flüssigen und festen Einsatzstoffe, einschließlich eventuell verwendeter Lösungsmittel.

Für Katalysatoren der Art a) können Selen oder Selenverbindungen, beispielsweise in Mengen von 0,1 bis 3 Gew.-%, vorzugsweise 0,1 bis 1 Gew.-%, eingesetzt werden. Schwefel oder Schwefelverbindungen können beispielsweise in Mengen von 1 bis 15 Gew.-%, vorzugsweise 1 bis 5 Gew.-%, eingesetzt werden. Die Selen- bzw. Schwefelmenge ist hierbei jeweils als Gehalt an Selen bzw. Schwefel berechnet.

Die als Komplexligand geeigneten Stickstoff- und/oder Phosphorverbindungen können beispielsweise in Mengen von 0,01 bis 10 Gew.-%, vorzugsweise in Mengen von 0,1 bis 5 Gew.-% verwendet werden.

Die aktivierenden Halogenide können beispielsweise in Mengen von 0,1 bis 5 Gew.-%, vorzugsweise in Mengen von 0,1 bis 1,5 Gew.-% eingesetzt werden, berechnet als Halogenidanteil der Halogenverbindungen.

Neben den oben beschriebenen Komponenten können die Katalysatoren der Arten a) und b) weitere Komponenten enthalten, z.B. inerte Trägermaterialien, Zeolithe oder Metalloxide, insbesondere Oxide der Metalle Eisen, Chrom, Molybdän, Nickel, Vanadin, Kobalt und Niob und basische oder puffernde Substanzen, wie Natriumacetat, Kaliumacetat und Phosphate bzw. Hydrogenphosphate der Alkalimetalle und des Ammoniums. Die Menge dieser zusätzlichen Komponenten kann z.B. insgesamt bis zu 10 Gew.-% ausmachen, vorzugsweise werden sie mit insgesamt bis 5 Gew.-% verwendet.

Urethanbildende Katalysatorsysteme, die auch für das erfindungsgemäße Verfahren geeignet sind, sind beispielsweise beschrieben in US-PS 3 895 054, US-PS 3 956 360, US-PS 4 170 708, DE-OS 29 08 250, DE-OS 28 38 754, US-PS 3 448 140, US-PS 3 454 620, US-PS 4 178 455, US-PS 4 339 592, EP-OS 0 000 815, J 54 084 550, J 54 022 339, DE-OS 28 03 432, DE-OS 28 19 826, DE-OS 29 03 950, DE-OS 29 08 251 und EP 0 000 563.

Bevorzugte Katalysatorsysteme sind in der DE-OS 29 03 950 und der DE-OS 28 19 826 beschrieben. Von diesen sind insbesondere solche auf der Basis von Palladium bevorzugt, die - gegebenenfalls zusätzlich - 0,1 bis 5 Gew.-% eines ein- oder mehrzähniges tertiären Amins, eines ein- oder mehrzähnigen tertiären Phosphins und/oder des Amins, das dem Reduktionsprodukt der jeweils eingesetzten Verbindung A entspricht, oder Gemische solcher Verbindungen bis zu 5 Gew.-% enthalten.

Das erfindungsgemäße Verfahren wird im allgemeinen bei erhöhter Temperatur und erhöhtem Druck durchgeführt, beispielsweise bei Temperaturen im Bereich 80 bis 220°C und Drucken im Bereich 20 bis 500 bar. Sofern die Einsatzmaterialien es zulassen wird vorzugsweise bei Temperaturen im Bereich 150 bis 200°C gearbeitet. Bei temperaturempfindlichen Einsatzmaterialien wird vorzugsweise bei 80 bis 150°C und mit entsprechend längerer Reaktionszeit gearbeitet. Der Druck liegt vorzugsweise im Bereich 50 bis 200 bar. Im allgemeinen beträgt die Reaktionszeit wenige Minuten bis einige Stunden, abhängig von den Substraten, der Katalysatormenge und den sonstigen Reaktionsbedingungen.

Das erfindungsgemäße Verfahren kann kontinuierlich oder diskontinuierlich durchgeführt werden. Als Reaktionsgefäße sind insbesondere Druckapparate mit guter Durchmischung geeignet. Beispielsweise kann eine diskontinuierliche Umsetzung in einem Rührautoklaven oder eine kontinuierliche Umsetzung in einer Kaskade von mehreren, beispielsweise 2 bis 5 gerührten Druckreaktoren oder Strahldüsenreaktoren durchgeführt werden. Es ist auch möglich, in einem Rührautoklaven nur den Gaseinsatz kontinuierlich ein- oder durchzuleiten und die flüssige oder fest/flüssige Reaktionsphase absatzweise zuzuführen.

Die Aufarbeitung der Reaktionsgemische kann so erfolgen, daß man diese zunächst entspannt und dann nach an sich bekannten und üblichen Verfahren, wie Destillation,

insbesondere Vakuum- oder Wasserdampfdestillation, das gebildete momomere aromatische Hydroxyurethan isoliert und anschließend gegebenenfalls einer Feinreinigung unterwirft, beispielsweise einer Kristallisation, Extraktion oder nochmaligen Destillation. Sind feste Katalysatorbestandteile vorhanden, so werden diese zweckmäßigerweise vor der sonstigen Aufarbeitung durch Filtration, Dekantieren oder Zentrifugieren abgetrennt und gegebenenfalls in die Reaktion zurückgeführt. Bei Verwendung der bevorzugten Katalysatorsysteme kann die Rückführung der Edelmetallkomponente besondere einfach bewerkstelligt werden, da der Edelmetallanteil überwiegend auf den festen Katalysatorbestandteilen absorbiert vorliegt.

Die als Verfahrensprodukte erhaltenen Hydroxyurethane können durch thermische oder katalytische Spaltung in Hydroxyisocyanate überführt und als solche z.B. zur Herstellung von Wirkstoffen oder speziellen Polymeren verwendet werden. Zahlreiche Umsetzungen, z.B. die Herstellung von N,N'-substituierten Harnstoffen lassen sich auch direkt aus Hydroxyurethanen, ohne die Isocyanatzwischenstufe, durchführen. Auf diese Weise kann der Weg über die schwierig zu handhabenden und nicht lagerbeständigen Hydroxyisocyanate vermieden werden. Auf die Schwierigkeiten bei der Herstellung von Hydroxyisocyanaten weisen beispielsweise K. Kurita, S. Matsuda und Y.Iwakura in Makromol. Chem. 182 (1981), 1327 hin. Da Hydroxyurethane verkappte Hydroxyisocyanate sind, stellen sie auch für Polymere verwendbare, potentiell bifunktionelle Verbindungen dar.

Hydroxyurethane können beispielsweise als Zwischenprodukte für Wirkstoffe gemäß DE-OS 2 334 607, DE-OS 15 68 138, DE-OS 21 08 975, US-PS 3 933 470, US-PS 3 914 265, US-PS 3 852 332 und gemäß Hsiao, Y.Y. und Bardos. T.J in J. Med. Chem. 16 (1973), 4, 391-394 verwendet werden, außerdem für die Herstellung von Polyurethanen entsprechend J 5 6103-222, sowie als Fotostabilisatoren nach J. 5 2150-630. Die Bedeutung des erfindungsgemäßen Verfahrens liegt auch darin, daß bisher nur auf unwirtschaftliche Weise herstellbare Produkte durch die nunmehr in guten Ausbeuten zugänglichen Hydroxyurethane billiger hergestellt werden oder überhaupt erstmals sinnvoll in technischem Maßstab produziert werden können.

Im Hinblick auf den eingangs geschilderten Stand der Technik ist es außerordentlich überraschend, daß das erfindungsgemäße Verfahren aufgefunden werden konnte. Insbesondere aus der EP-OS 0 000 563 und der US-PS 3 956 360 war zu erwarten, daß die im Einsatzprodukt A enthaltenen Hydroxygruppen unter den erfindungsgemäßen Reaktionsbedingungen reagieren und nicht erhalten bleiben.

Gegenüber der EP-OS 94 861 ist überraschend, daß mit Katalysatorzusätzen, die dort die Polyurethanbildung fördern, beim erfindungsgemäßen Verfahren in besseren Ausbeuten, beim Einsatz geringerer Mengen an Edelmetall-Katalysatoren monomere Hydroxyurethane ohne fremdes Lösungsmittel und ohne große Edelmetallverluste in guten Ausbeuten erhalten werden können.

Die nachstehenden Beispiele illustrieren das erfindungsgemäße Verfahren, ohne es in irgendeiner Weise einzuschränken.

Beispiele

Allgemeine Arbeitsweise:

Alle Beispiele wurden unter Rühren in 0,7 l Autoklaven - soweit nicht anders angegeben in Edelstahlautoklaven - durchgeführt. Die Einwaage an organischer Hydroxyverbindung betrug, falls nichts anderes angegeben ist, jeweils 200 g. Die Einsatzmengen werden im übrigen in Gew.-%, bezogen auf die Gesamtmenge bei Normalbedingungen flüssigen und festen Einsatzprodukte, angegeben. Eingesetzte Edelmetallverbindungen sind entsprechend in Gew.-ppm angegeben. Die beschickten Autoklaven wurden zunächst mit Stickstoff gespült, dann wurden bei Raumtemperatur 120 bar Kohlenmonoxid aufgepreßt. Unter Rühren wurde innerhalb 1/2 Stunde auf Reaktionstemperatur geheizt. Falls nichts anderes angegeben ist, betrug die Reaktionszeit 2 Stunden und die Reaktionstemperatur 180°C. Nach dem Abkühlen wurde entspannt, die Reaktionsphase ausgewogen und durch Gaschromatographie und/oder Hochdruckflüssigkeitschromatographie quantitativ analysiert. Die Selektivität, in der das Hydroxyurethan gebildet wurde, ist in Mol-%, bezogen auf eingesetzte Verbindung A angegeben.

Beispiel 1 (zum Vergleich)

Einsatz: 85,4 % Ethanol, 12,0 % 4-Nitrophenol, 1,3 % Pyridin und 1,3 % Aktivkohle beladen mit 1 % Palladium (bezogen auf Aktivkohle).

0153642

Umsatz von 4-Nitrophenol: 10 %
Selektivität zu 4-Hydroxyphenylcarbaminsäureethylester (4-HCE): 21 %

Beispiel 2

Einsatz: 83,9 % Ethanol, 11,8 % 4-Nitrophenol, 1,7 % $FeCl_2$ x 2,2 $H_2O$ 1,3 % Pyridin, 1,3 % Aktivkohle beladen mit 1 % Palladium (bezogen auf Aktivkohle).
Umsatz von 4-Nitrophenol: 47 %
Selektivität zu 4-HCE: 60 %.

Beispiel 3

Einsatz: 85,1 % Ethanol, 11,9 % 4-Nitrophenol, 1,7 % $FeCl_2$ x 2,2 $H_2O$, 1,3 % Pyridin, 43 ppm $PdCl_2$.
Umsatz von 4-Nitrophenol: 100 %
Selektivität zu 4-HCE: 91 %.

Beispiel 4

Einsatz: 82,4 % Ethanol, 11,5 % 4-Nitrophenol, 3,3 % $\alpha$-$Fe_2O_3$, 1,6 % $FeCl_2$ x 2,2 $H_2O$, 1,2 % Pyridin, 41 ppm $PdCl_2$; Reaktionszeit 1 Stunde.
Umsatz von 4-Nitrophenol: 89 %
Selektivität zu 4-HCE: 85 %.

Beispiel 5

Einsatz: Wie bei Beispiel 4, Reaktionszeit jedoch 4 Stunden bei 120°C.

Umsatz von 4-Nitrophenol: 100 %
Selektivität zu 4-HCE: 74 %.

Beispiel 6

Einsatz: 84,4 % Ethanol, 11,8 % 4-Nitrophenol, 1,3 % Pyridin, 2,5 % $VOCl_3$, 42 ppm $PdCl_2$.
Umsatz von 4-Nitrophenol: 100 %
Selektivität zu 4-HCE: 80 %.

Beispiel 7

Einsatz: Wie bei Beispiel 6, jedoch wurden bei Raumtemperatur 100 bar Kohlenmonoxid und 20 bar Wasserstoff aufgepreßt. Die Reaktionszeit betrug 2 Stunden bei 180°C.
Umsatz von 4-Nitrophenol: 100 %
Selektivität zu 4-HCE: 75 %.

Beispiel 8

Einsatz: 85,1 % Ethanol, 11,9 % 4-Nitrophenol, 1,3 % 1,4-Diazabicyclo[2,2,2]octan, 0,4 % Selen (metallisch) und 1,3 % Anilin.
Umsatz von 4-Nitrophenol: 100 %
Selektivität zu 4-HCE: 48 %.

Beispiel 9

Einsatz: 82,9 % Ethanol, 11,6 % 4-Nitrophenol, 0,8 %

Schwefel, 1,3 % Anilin, 1,0 % Kaliumacetat, 1,1 % $\alpha$-$Fe_2O_3$, 0,2 % $V_2O_5$, 1,1 % Di-n-butylamin.
Umsatz von 4-Nitrophenol: 96 %
Selektivität zu 4-HCE: 52 %.

Beispiele 10 bis 17

In den Beispielen 10 bis 13 wurde keine Katalysatorkomponente basierend auf Schwefel, Selen oder einem Metall der 8. Nebengruppe des periodischen Systems zugesetzt, jedoch enthielt die Autoklavenwand von vorhergehenden Versuchen (siehe Beispiele 3 bis 7) noch Palladium, so daß in den aufeinanderfolgend durchgeführten Beispielen 10 bis 17 bis Beispiel 13 eine Abnahme von Umsatz und Selektivität und erst bei erneutem Edelmetallzusatz bei den Beispielen 14 bis 17 wieder eine Zunahme zu beobachten war.

Der Einsatz für die Beispiele 10 bis 17 betrug:
82,4 % Ethanol, 11,5 % 4-Nitrophenol, 3,3 % $\alpha$-$Fe_2O_3$, 1,6 % $FeCl_2$ x 2,2 $H_2O$, 1,2 % Pyridin.
In die Beispiele 14 bis 17 wurden zusätzlich 41 ppm eines Edelmetallchlorids eingesetzt (siehe Tabelle 2).

Die Ergebnisse sind aus den Tabellen 1 und 2 ersichtlich.

Tabelle 1

| Beispiel | 10 | 11 | 12 | 13 |
|---|---|---|---|---|
| Umsatz von 4-Nitrophenol (%) | 47 | 39 | 2 | 1 |
| Selektivität zu 4-HCE (%) | 95 | 84 | 5 | - |

Tabelle 2

| Beispiel | 14 | 15 | 16 | 17 |
|---|---|---|---|---|
| Edelmetallchlorid | $RuCl_3$ | $RhCl_3$ | $IrCl_3$ | $PtCl_2$ |
| Umsatz von 4-Nitrophenol (%) | 12 | 16 | 27 | 13 |
| Selektivität zu 4-HCE | 20 | 38 | 6 | 8 |

Beispiele 18 bis 32

Einsatz: 82,5 % Ethanol, 11,4 % 4-Nitrophenol, 3,3 % $\alpha$-$Fe_2O_3$, 1,6 % $FeCl_2$ x 2,2 $H_2O$, 41 ppm $PdCl_2$, sowie 1,2 % einer als Komplexligand geeigneten Stickstoff- oder Phosphorverbindung. Die Ergebnisse sind aus Tabelle 3 ersichtlich.

Tabelle 3

| Beispiel | N- oder P-Verbindung | Umsatz von 4-Nitrophenol (%) | Selektivität zu 4-HCE (%) |
|---|---|---|---|
| 18 | p-Aminophenolhydrochlorid | 86 | 90 |
| 19 | p-Aminophenol | 86 | 95 |
| 20 | Chinolin | 67 | 95 |
| 21 | Imidazol | 95 | 96 |
| 22 | Benzimidazol | 82 | 96 |
| 23 | $(H_3C)_2N-CH_2-CH_2-N(CH_3)_2$ | 60 | 83 |
| 24 | Anilin | 85 | 65 |
| 25 | N-Ethylanilin | 71 | 95 |
| 26 | N,N-Diethylanilin | 90 | 84 |
| 27 | Triethylamin | 52 | 83 |
| 28 | Pyro-PAN *) | 95 | 85 |
| 29 | Triphenylphosphin | 88 | 75 |
| 30 | Triphenylphosphit | 37 | 59 |
| 31 | $(PH)_2P-CH_2-CH_2-P(PH)_2$ **) | 15 | 13 |
| 32 | $(PH)_2P-(CH_2)_3-P(PH)_2$ **) | 33 | 26 |

*) pyrolisiertes Polyacrylnitril hergestellt nach J. Manassen und Sh. Khalif, J. Am. Chem. Soc. 88, 1943 (1966).

**) PH steht für Phenyl.

Beispiele 33 bis 40

Einsatz: 82,4 % (= 200 g) einer organischen Hydroxylverbindung, 11,5 % 4-Nitrophenol, 3,3 % $\alpha$-$Fe_2O_3$, 1,6 % $FeCl_2$ x 2,2 $H_2O$, 1,2 % Pyridin, 41 ppm $PdCl_2$. Die Ergebnisse sind aus Tabelle 4 ersichtlich.

Tabelle 4

| Beispiel | organische Hydroxyverbindung | Umsatz von 4-Nitrophenol (%) | Selektivität zu 4-Hydroxyphenylcarbaminsäureester (%) |
|---|---|---|---|
| 33 | $CH_3OH$ | 100 | 91 |
| 34 | $C_2H_5OH$ | 100 | 94 |
| 35 | $CH_3-CH_2-CH_2OH$ | 100 | 88 |
| 36 | $(CH_3)_2CHOH$ | 100 | 70 |
| 37 | $(CH_3)_3C-CH_2OH$ | 89 | 87 |
| 38 | Cyclohexanol | 100 | 82 |
| 39 | Benzylalkohol | 64 | 96 |
| 40 | Diethylenglykolmonomethylether | 74 | 95 |

Beispiel 41

Einsatz: 9,9 % (21,3 g) 3-Hydroxypropannitril, 70,1 % ortho-Dichlorbenzol, 13,0 % 4-Nitrophenol, 3,7 % α-$Fe_2O_3$, 1,9 % $FeCl_2$ x 2,2 $H_2O$, 1,4 % Pyridin, 47 ppm $PdCl_2$.
Umsatz von 4-Nitrophenol: 5 %
Selektivität zu 4-Hydroxyphenylcarbaminsäure-2-cyanoethylester: 90 %.

Beispiel 42

Einsatz: 8,3 % (17,4 g) Allylalkohol, 71,3 % Aceton, 13,3 % 4-Nitrophenol, 3,8 % α-$Fe_2O_3$, 1,9 % $FeCl_2$ x 2,2 $H_2O$, 1,4 % Pyridin, 47 ppm $PdCl_2$.
Reaktionsbedingungen: 4 Stunden bei 150°C.
Umsatz von 4-Nitrophenol: 95 %
Selektivität zu 4-Hydroxyphenylcarbaminsäureallylester: 82 %.

Beispiele 43 bis 51

Einsatz: 200 g Ethanol, 0,2 Mol Verbindung A, 8 g α-$Fe_2O_3$, 4 g $FeCl_2$ x 2,2 $H_2O$, 3 g Pyridin, 10 mg $PdCl_2$.
Die Ergebnisse sind aus der Tabelle 5 ersichtlich.

Tabelle 5

| Beispiel | Verbindung A | Umsatz von Verbindung A (%) | Selektivität zu (substituiertem) Hydroxyphenylethylcarbamat (%) |
|---|---|---|---|
| 43 | 2-Chlor-4-nitrophenol | 92 | 96 |
| 44 | 3-Chlor-4-nitrophenol | 90 | 82 |
| 45 | 3-Nitrophenol | 95 | 88 |
| 46 | 4-Methyl-3-nitrophenol | 96 | 94 |
| 47 | 4-Chlor-3-nitrophenol | 99 | 85 |
| 48 | 2-Nitrophenol | 100 | 8 |
| 49 | 2,4-Dichlor-5-nitro-phenol | 100 | 60 |
| 50 | 4-Nitrosophenol | 100 | 94 |
| 51 | 4,4'-Dihydroxyazobenzol | 100 | 96 |

Beispiel 52

Einsatz: 20,6 % Phenol (50 g), 61,8 % Toluol, 11,5 % 4-Nitrophenol, 3,3 % $\alpha$-$Fe_2O_3$, 1,6 % $FeCl_2$ x 2,2 $H_2O$, 1,2 % Pyridinhydrochlorid und 41 ppm $PdCl_2$. Nach dem Aufpressen von 120 bar CO wurde 2 Stunden bei 180°C umgesetzt.

Das erkaltete, zu einen Kristallkuchen erstarrte Reaktionsgemisch wurde auf einer Nutsche trocken gesaugt und dann mit 200 ml warmen Methylenchlorid behandelt, um die organischen Produkte zu lösen. Nach Abfiltrieren der unlöslichen Katalysatorbestandteile wurde die Methylenchloridphase zweimal mit Natriumhydrogencarbonatlösung gewaschen, über Natriumsulfat getrocknet und bei Raumtemperatur (Vakuum bis 10 mbar) zur Trockene eingeengt. Der Rückstand (24 g) wurde aus Chloroform umkristallisiert und ergab 16 g p-Hydroxyphenylcarbaminsäurephenylester mit einem Schmelzpunkt von 152-153°C. Schmelzpunkt, IR- und NMR-Daten stimmten mit den Angaben von K. Kurita, S. Matsuda und I. Iwakura in Makromol. Chem. 182 (1981), 1327-1335 auf S. 1328 überein.

Beispiele 53 - 58

Die nachfolgenden Beispiele 53 - 58 wurden in der angegebenen Reihenfolge nacheinander in demselben 0,7 l-Hastelloy C-Autoklaven durchgeführt.

Beispiel 53 (zum Vergleich)

Im Autoklaven wurden 14 g 4-Nitrophenol (0,1 Mol) und 9,24 g Ethanol (0,2 Mol) vorgelegt; das Molverhältnis Ethanol/Nitrobenzol war somit 2. Das Katalysatorsystem, bestehend aus 0,588 g $PdCl_2$, 0,490 g $MoO_3$ und 0,490 Mol Pyridin, wurde hinzugefügt; das Molverhältnis Pd/Nitrophenol betrug somit $3,3 \cdot 10^{-2}$. Man füllte mit 116 ml (entsprechend 150,6 g) ortho-Dichlorbenzol auf, spülte mit Stickstoff, drückte 200 bar Kohlenmonoxid auf und heizte unter Rühren innerhalb von 30 min. auf 200°C. Nach dem Abkühlen wurde der Autoklav entleert und mit 200 ml Aceton nachgespült. Der Katalysator wurde abfiltriert, mit Aceton nachgewaschen und bei 10 mbar bei 50°C getrocknet.

Das Filtrat wurde mit allen Acetonphasen vereint und gaschromatographisch analysiert; die Analyse ergab einen Umsatz von 28 % für 4-Nitrophenol und eine Selektivität von 69 Mol-% für 4-HCE. Nach Einengen der organischen Phase zur Trockene am Rotationsverdampfer im Wasserstrahlvakuum bei maximal 80°C Badtemperatur wurden 16,45 g Rückstand erhalten, dessen Hauptbestandteil nach GC-Analyse 4-Nitrophenol war. Durch Umkristallisieren aus Wasser wurden daraus 2,41 g 4-Nitrophenol vom Schmp. 111°C erhalten.

Der zurückgewonnene Katalysator (0,93 g) enthielt 29,6 % Pd, bestimmt durch Atomabsorptions-Spektroskopie, dies entsprach 78 % der eingesetzten Palladiummenge, d.h. 22 % des eingesetzten Palladiums gingen verloren.

Beispiel 54

| | | | |
|---|---|---|---|
| Einsatz: | 200 g | Ethanol | 82,30 Gew.-% |
| | 28 g | 4-Nitrophenol | 11,52 Gew.-% |
| | 8 g | α-$Fe_2O_3$ | 3,29 Gew.-% |
| | 4 g | $FeCl_2 \cdot 2,2\ H_2O$ | 1,65 Gew.-% |
| | 3 g | Pyridin | 1,23 Gew.-% |

Nach Aufdrücken von 120 bar CO wurde 2 Std. bei 180°C umgesetzt. Die Analyse des Reaktionsgemisches zeigte einen Umsatz des 4-Nitrophenols von 96 % bei 92 Mol-% Selektivität für 4-HCE.

Das Beispiel belegt, daß für das erfindungsgemäße Verfahren äußerst geringe Pd-Mengen genügen, da hier nur das von dem Vorversuch in der Autoklavenwand verbliebene Pd für die erfindungsgemäße Umsetzung zur Verfügung stand und durch das Katalysatorsystem aktiviert wurde.

Beispiel 55 (zum Vergleich)

Es wurde wie in Beispiel 53 gearbeitet, jedoch wurden nur 5 mg $PdCl_2$ ( ≙ 17 ppm Pd) eingesetzt; dies entsprach einem Molverhältnis Pd/Nitrophenol von $2,8 \cdot 10^{-4}$.
Umsatz von 4-Nitrophenol: 9 %
Selektivität zu 4-HCE: 70 %

Beispiel 56 (zum Vergleich)

Es wurde wie in Beispiel 55 gearbeitet, jedoch wurden nur 120 bar CO aufgepreßt und 2 Std. bei 180°C umgesetzt.
Umsatz von 4-Nitrophenol: 5 %
Selektivität zu 4-HCE: 70 %

Beispiel 57 zum Vergleich)

Es wurde wie in Beispiel 56 gearbeitet, jedoch wurde anstelle von ortho-Dichlorbenzol das gleiche Volumen an Ethanol eingesetzt.
Umsatz von 4-Nitrophenol: 17 %
Selektivität zu 4-HCE: 65 %

Beispiel 58

Es wurde wie in Beispiel 54 gearbeitet, jedoch wurden zusätzlich 10 mg $PdCl_2$ ( ≙ 25 ppm Pd) eingesetzt; dies entsprach einem Molverhältnis Pd/Nitrophenol von $2{,}8 \cdot 10^{-4}$.
Umsatz von 4-Nitrophenol: 100 %
Selektivität zu 4-HCE: 94 %

0153642

Patentansprüche

1) Verfahren zur Herstellung monomerer aromatischer Hydroxyurethane, dadurch gekennzeichnet, daß man eine aromatische Verbindung, die pro Molekül mindestens eine direkt an einen aromatischen Kern gebundene Hydroxylgruppe und mindestens eine Nitro-, Nitroso-, Azo- oder Azoxygruppe enthält, mit einer organischen Hydroxyverbindung und Kohlenmonoxid in Gegenwart eines Katalysators umsetzt, der a) Schwefel und/oder Selen oder b) Edelmetalle der 8. Nebengruppe des Periodensystems der Elemente in elementarer und/oder in Form von Verbindungen, eine oder mehrere als Komplexligand geeignete Stickstoff- und/oder Phosphorverbindungen und eines oder mehrere aktivierende Halogenide enthält.

2) Verfahren nach Anspruch 1, dadurch gekennzeichnet, daß man eine Nitro- oder Nitrosoverbindung der Formel (I) oder eine Azo- oder Azoxyverbindung der Formel (II)

$$(HO)_a Z(NO_x)_b \qquad \text{(I)}$$

$$(HO)_{a}\underset{(NO_x)_c}{Z}-[\bar{N}=\overset{(O)_d}{N}-R_1]_b \qquad \text{(II)}$$

einsetzt, in denen

Z für einen insgesamt 4 bis 30 C-Atome enthaltenden Rest steht, der mindestens einen aromatischen Ring enthält und der gegebenenfalls weitere als die in den Formeln (I) und (II) angegebenen Substituenten enthält,

x für 1 oder 2 steht,

a und b unabhängig voneinander für 1, 2, 3 oder 4 stehen,

c für Null, 1, 2 oder 3 steht,

die Summe a + b + c jedoch nicht größer ist, als die um 2 verminderte Anzahl von substituierbaren Stellen an Z

d für Null oder 1 steht,

$R_1$ für einen aliphatischen, cycloaliphatischen oder aromatischen Rest mit insgesamt 1 bis 30 C-Atomen steht, der gegebenenfalls substituiert sein kann,

und wobei mindestens eine HO-Gruppe direkt an einen aromatischen Ring von Z gebunden ist.

3) Verfahren nach Anspruch 2, dadurch gekennzeichnet, daß man eine Nitrosoverbindung der Formel (I) oder

eine Azo- oder Azoxyverbindung der Formel (II) einsetzt, in der Z für einen Rest steht, der das Grundgerüst von Benzol, Naphthalin oder Pyridin als Grundstruktur enthält und gegebenenfalls mit bis zu 4 gleichen oder verschiedenen Substituenten aus der Gruppe Chlor, Fluor, Methyl, Methoxy, Ethoxy, Isopropoxy, N,N-Dimethylamino, N-Acetylamino und Carboxy-$C_1$ bis $C_4$-alkyl substituiert ist.

4) Verfahren nach Ansprüchen 1 bis 3, dadurch gekennzeichnet, daß man als organische Hydroxyverbindung eine Verbindung der Formel (III)

$$R_2-(OH)_n$$

einsetzt, in der

$R_2$ für einen gegebenenfalls substituierten organischen Rest mit insgesamt 1 bis 30 C-Atomen und

n für 1, 2, 3 oder 4 steht.

5) Verfahren nach Anspruch 4, dadurch gekennzeichnet, daß man eine organische Hydroxyverbindung der Formel (III) einsetzt, in der $R_2$ für einen gegebenenfalls substituierten organischen Rest mit insgesamt 1 bis 12 C-Atomen und n für 1 oder 2 steht.

6) Verfahren nach Ansprüchen 1 bis 5, dadurch gekennzeichnet, daß das Kohlenmonoxid bis zu 70 Vol.-% Stickstoff, Helium, Argon, Kohlendioxid und/oder niedere Kohlenwasserstoffe und/oder bis zu 5 Vol.-% Wasserstoff und/oder Ammoniak enthält.

7) Verfahren nach Ansprüchen 1 bis 6, dadurch gekennzeichnet, daß der Katalysator a) elementaren Schwefel, Carbonylsulfid, Schwefelwasserstoff, Alkalisulfid, Dimethylsulfid, Thiophen, Thioharnstoff, metallisches Selen, Selendioxid, Carbonylselenid, Dimethylselenid oder Diphenylselenid und der Katalysator b) Ruthenium, Rhodium, Palladium, Osmium, Iridium oder Platin in elementarer Form oder in Form von Verbindungen, ein primäres, sekundäres oder tertiäres, lineares oder heterocyclisches Amin, einen Harnstoff, ein Biuret oder ein substituiertes Phosphin oder Phosphit und ein Halogenid und/oder Oxyhalogenid eines Metalles, das in mehreren Oxidationsstufen auftreten kann, oder Lithiumchlorid, Ammoniumchlorid, substituiertes Ammoniumchlorid oder Chlorwasserstoff enthält.

8) Verfahren nach Ansprüchen 1 bis 7, dadurch gekennzeichnet, daß der Katalysator a) 1 bis 15 Gew.-% Schwefel oder Schwefelverbindungen oder 0,1 bis 3 Gew.-% Selen oder Selenverbindungen und der Katalysator b) 0,0001 bis 0,02 Gew.-% Edelmetalle der 8. Nebengruppe des Periodensystems der Elemente oder Verbindungen davon, 0,01 bis 10 Gew.-% einer als Komplexligand geeigneten Stickstoff- und/oder Phosphorverbindung und 0,1 bis 5 Gew.-% eines aktivierenden Halogenids enthält, wobei sich im Falle von Schwefel-, Selen-, Edelmetall- oder Halogenidverbindungen die

angegebenen Gew.-% auf deren Gehalt an Schwefel, Selen, Edelmetall bzw. Halogenid beziehen und sich sämtliche angegebenen Gew.-% auf die Gesamtmenge der bei Normalbedingungen flüssigen unf festen Einsatzstoffe beziehen.

9) Verfahren nach Ansprüchen 1 bis 8, dadurch gekennzeichnet, daß der Katalysator zusätzlich inerte Trägermaterialien, Zeolithe, Metalloxide, basische Substanzen und/oder puffernde Substanzen enthält.

10) Verfahren nach Ansprüchen 1 bis 9, dadurch gekennzeichnet, daß man es bei Temperaturen im Bereich 80 bis 220°C und Drucken im Bereich 20 bis 500 bar durchführt.